# EUROPEAN PATENT APPLICATION

(11) **EP 3 933 848 A1**
(43) Date of publication of application: **05.01.2022**
(21) Application number: 19917170.3
(22) Date of filing: 16.08.2019
(51) Int. Cl.: G16H 30/20, G06T 7/00

(54) **VRDS 4D MEDICAL IMAGE PROCESSING METHOD AND PRODUCT**

(30) Priority: 28.02.2019 CN 201910153010
(71) Applicant: VR Doctor Medical Technology (Shenzhen) Co., Ltd., Shenzhen, Guangdong, 518035 (CN)
(72) Inventor: LEE, Stewart Ping, Shenzhen, Guangdong 518035 (CN); LEE, David Wei, Shenzhen, Guangdong 518035 (CN)
(74) Representative: Dargiewicz, Joanna
(86) International application number: PCT/CN2019/101161
(87) International publication number: WO 2020/173054

(57) **Abstract**

Disclosed in embodiments of the present application are a method and a product for processing of VRDS 4D medical images, the method is applied to a medical imaging apparatus, and the method includes: performing initial data analysis processing on a plurality of scanned images to obtain an image source including image features of a target site of a target user; performing post-data processing on the image source to obtain N first image data sets; performing preset processing on the N first image data sets to obtain N second image data sets; performing VRDS 4D medical image display according to the N second image data sets. The embodiment of this application is facilitated to improve the refinement degree and accuracy of the medical imaging apparatus in performing medical image display.

## Description

### TECHNICAL FIELD

This application relates to the field of medical imaging apparatus, and in particular, to a method and a product for processing of VRDS 4D medical images.

### BACKGROUND

In current, doctors use technologies such as Computed Tomography (CT), Magnetic Resonance Imaging (MRI), Diffusion Tensor Imaging (DTI), Positron Emission Computed Tomography (PET) to acquire information such as the shape, position and topology of the pathological tissue. Doctors still use watching to read continuous two-dimensional slice scanned images, so as to perform judging and analyzing on the pathological tissue of the patients such as tumors. However, the two-dimensional slice scanned image can not present the spatial structure characteristics of pathological tissue, which affects the diagnosis of the doctors on the diseases. With rapid development of medical imaging technology, people put forward new demands for medical imaging.

### SUMMARY

Embodiments of this application provide a method and a product for processing of VRDS 4D medical images, in order to improve the refinement degree and accuracy of the medical imaging apparatus in performing medical image display.

In a first aspect, embodiments of this application provide a method for processing of VRDS 4D medical images, the method is applied to a medical imaging apparatus; and the method includes:
performing initial data analysis processing on a plurality of scanned images to obtain an image source including image features of a target site of a target user, wherein the image source includes Texture 2D/3D image volume data, and the plurality of scanned images are two-dimensional medical images collected by a medical device;
performing post-data processing on the image source to obtain N first image data sets, wherein a data amount of each first image data set is the same as that of the image source, and data in any two first image data sets are independent from each other, and N is a positive integer greater than 1;
performing preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data;
and performing VRDS 4D medical image display according to the N second image data sets.

In a second aspect, embodiments of this application provide an apparatus for processing of VRDS 4D medical images, the apparatus is applied to a medical imaging apparatus; the apparatus for processing of VRDS 4D medical image includes a processing unit and a communication unit, wherein,
the processing unit is configured to: perform initial data analysis processing on a plurality of scanned images to obtain an image source including image features of a target site of a target user, wherein the image source includes Texture 2D/3D image volume data, and the plurality of scanned images are two-dimensional medical images collected by a medical device; perform post data processing on the image source to obtain N first image data sets, wherein a data amount of each first image data set is the same as that of the image source, and the data in any two first image data sets are independent from each other, and N is a positive integer greater than 1; perform preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and perform VRDS 4D medical image display according to the N second image data sets through the communication unit.

In a third aspect, embodiments of this application provide an medical imaging apparatus, the apparatus includes a processor, a memory, a communication interface, and one or more programs, wherein the above one or more programs are stored in the above memory and configured to be executed by the above processor, and the above programs include instructions for executing the steps in any method of the first aspect of the embodiment of this application.

In a fourth aspect, embodiments of this application provide a computer-readable storage medium, wherein the above computer-readable storage medium stores a computer program for electronic data exchange, wherein the above computer program causes a computer to execute some or all of the steps described in any methods of the first aspect of the embodiment of this application.

In a fifth aspect, embodiments of this application provide a computer program product, wherein the above computer program product includes a non-transitory computer-readable storage medium in which a computer program is stored, the above computer program is operable to cause the computer to execute some or all of the steps described in any methods of the first aspect of the embodiment of this application. The computer program product can be a software installation package.

As can be seen that in the embodiment of this application, first, the processing unit performs initial data analysis processing on a plurality of scanned images to obtain an image source including image features of a target site of a target user; second, performs post data processing on the image source to obtain N first image data sets, wherein a data amount of each first image data set is the same as that of the image source, and the data in any two first image data sets are independent from each other, and N is a positive integer greater than 1; third, performs preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and finally, performs VRDS 4D medical image display according to the N second image data sets. It can be seen that the medical imaging apparatus in this application can perform data optimization on the raw scanned image in the data volume dimension and spatial dimension, so as to achieve VRDS 4D medical image display, that is, to present 3D images outside and inside the tissue with real spatial structure characteristics, and to improve the refinement degree and accuracy of the medical imaging apparatus in performing medical image display.

### BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.
Fig. 1 is a schematic structural diagram of a medical image intelligent analysis and processing system based on VRDS 4D provided by an embodiment of this application;
Fig. 2 is a schematic flowchart of a method for processing of VRDS 4D medical images provided by an embodiment of this application;
Fig. 3 is a schematic structural diagram of an medical imaging apparatus provided by an embodiment of this application;
Fig. 4 is a block diagram of functional units composition of an apparatus for processing of VRDS 4D medical images provided by an embodiment of this application.

### DETAILED DESCRIPTION OF PREFERRED EMBODIMENTS

In order to make person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "have" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the person skilled in the art that the described embodiment may be combined with other embodiments.

The medical imaging apparatuses related to the embodiments of this application refer to various instruments that use various different media as information carriers to reproduce the internal structure of the human body as images, their image information has a spatial and temporal distribution corresponding relationship with the actual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET-CT), "image source" refers to Texture 2D/3D image volume data generated by parsing the raw DICOM data. "VRDS" refers to Virtual Reality Doctor system.

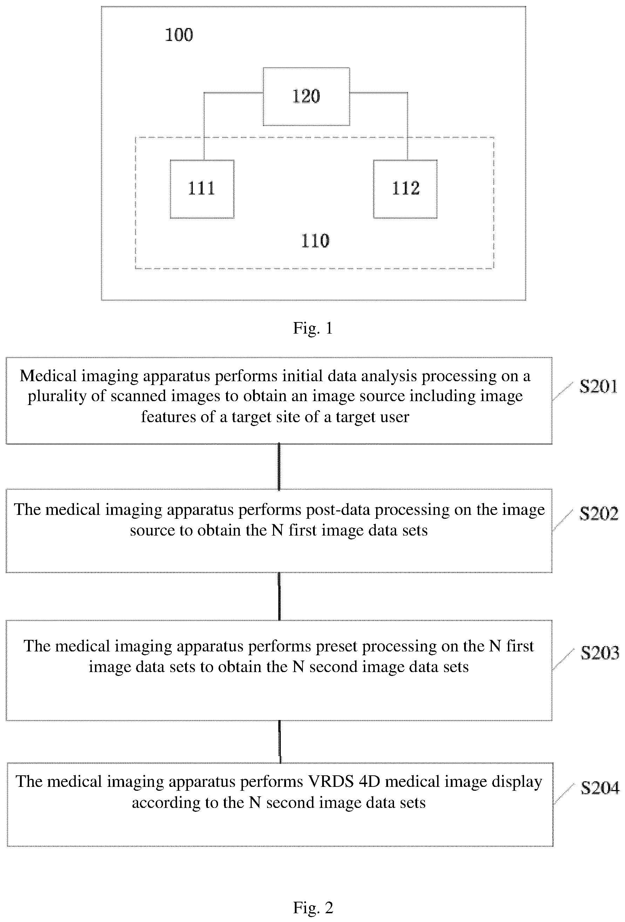

Referring to Fig. 1, Fig. 1 is a schematic structural diagram of a intelligent analyzing and processing system 100 based on VRDS 4D medical image provided by an embodiment of this application, the system 100 includes a medical imaging apparatus 110 and a network database 120, wherein the medical imaging apparatus 110 may include a local medical imaging apparatus 111 and/or a terminal medical imaging apparatus 112, the local medical imaging apparatus 111 or the terminal medical imaging apparatus 112 is configured to perform four-dimensional 4D volume drawing on the occupation of tissues and organs inside the human body based on the raw DICOM data and the processing algorithm based on VRDS 4D medical image presented in the embodiment of this application, so as to achieve the 4D stereo effect (the 4-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.), compared with the terminal medical imaging apparatus 112, the local medical imaging apparatus 111 can further configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the above medical imaging apparatus 110, the user can choose a display and/or a head mounted displays set (HMDS) of virtual reality VR to display in combination with operation actions, which refer to the operation control of the four-dimensional human body image by the user through external intake device of the medical imaging apparatus, such as a mouse and a keyboard, so as to achieve human-computer interaction, the operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and the shearing effect rendering in real time, and (5) moving the view up and down.

The following describes the tumor recognition algorithm based on VRDS 4D medical image in detail.

Referring to Fig. 2, Fig. 2 is a schematic flowchart for processing of VRDS 4D medical images provided by an embodiment of this application, which is applied to medical imaging apparatus as described in Fig. 1; as shown in the Figure, the method for processing of VRDS 4D medical images includes:
S201, performing, by a medical imaging apparatus, initial data analysis processing on a plurality of scanned images to obtain an image source including image features of a target site of a target user, wherein the image source includes Texture 2D/3D image volume data, and the plurality of scanned images are two-dimensional medical images collected by a medical device.

Wherein, the target site may include organs such as kidneys, and arteries, veins, etc. And the a plurality of scanned image includes any one of the following: a CT image, an MRI image, a DTI image and a PET-CT image.

In a specific implementation, the medical imaging apparatus acquires a plurality of scanned images collected by a medical device and reflecting internal structure features of human body of the target user; screens at least one scanned image including the target site from the plurality of scanned images, and takes the at least one scanned image as medical digital imaging and communication DICOM data of the target user; parses the DICOM data to generate a image source of the target user, wherein the image source includes Texture 2D/3D image volume data.

S202, the medical imaging apparatus performs post-data processing on the image source to obtain N first image data sets, wherein a data amount of each first image data set is the same as that of the image source, and data in any two first image data sets are independent from each other, and N is a positive integer greater than 1;

Wherein, the medical imaging device performs data enhancement processing on the map source, the data enhancement processing includes at least one of the following: data enhancement based on arbitrary angle rotation, data enhancement based on scaling, data enhancement based on translation, data enhancement based on mirror operation, data enhancement based on shearing and data enhancement based on elastic deformation.

Wherein, the image rotation refers to the geometric transformation of rotating the image source by a certain angle; the scaling refers to the process of adjusting the size of digital images in computer image processing, that is, making a trade-off between processing efficiency and smoothness and sharpness of results; the translation is to add the specified horizontal offset and vertical offset to all pixel coordinates of the image. the shearing can be divided into two types: Rectangle Subset and Pdygon Subset, Rectangle Subset means that the boundary range of the clipped image is a rectangle, and the clipping position of the image can be determined by the coordinates of the upper left corner and the lower right corner, the whole clipping process is relatively simple, and Pdygon Subset means that the boundary range of the clipped image is an arbitrary polygon, the mirror image is divided into two types: horizontal mirroring and vertical mirroring. Horizontal mirroring takes the vertical midline of the image as the axis, switching pixels of the image, that is, switching the left half and the right half of the image, while vertical mirroring takes the horizontal midline of the image as the axis, switching the upper half and the off-duty part of the image.

S203, the medical imaging apparatus performs preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data.

Wherein, performing preset processing on the N first image data sets includes data separation, integration and the like. The spatial position information includes spatial direction information, spatial topology information and spatial metric information.

S204, the medical imaging apparatus performs VRDS 4D medical image display according to the N second image data sets.

Wherein, the VRDS 4D medical image display refers to presenting VRDS four-dimensional medical images.

Wherein, the medical imaging apparatus screens out target image data from the N second image data sets as VRDS 4D imaging data.

As can be seen that in the embodiment of this application, first, the processing unit performs initial data analysis processing on a plurality of scanned images to obtain an image source including image features of a target site of a target user; second, performs post data processing on the image source to obtain N first image data sets, wherein a data amount of each first image data set is the same as that of the image source, and the data in any two first image data sets are independent from each other, and N is a positive integer greater than 1; third, performs preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and finally, performs VRDS 4D medical image display according to the N second image data sets. It can be seen that the medical imaging apparatus in this application can perform data optimization on the raw scanned image in the data volume dimension and spatial dimension, so as to achieve VRDS 4D medical image display, that is, to present 3D images outside and inside the tissue with real spatial structure characteristics, and to improve the refinement degree and accuracy of the medical imaging apparatus in performing medical image display.

In one possible example, the performing of the post-data processing on the image source to obtain the N first image data sets, including: performing data enhancement processing on the image source to obtain N first image data sets, wherein the data enhancement processing includes at least one of the following: rotation, scaling, translation, shearing, mirroring and elastic deformation.

In a specific implementation, in one data enhancement process, the enhancement processing ways for all data in the image source include at least one of the following: rotation, scaling, translation, shearing, mirroring and elastic deformation; taking the data after the data enhancement processing as one first image data set; in N times of data enhancement processing, N first image data sets can be obtained according to the image source data and various data enhancement processing ways.

It can be seen that in this example, because the medical imaging apparatus can perform data enhancement processing on image sources based on image data processing ways such as rotation, scaling, translation, cutting, mirroring, and elastic deformation to obtain N first image data sets, the image quality in the first image data sets is improved, and the clarity and accuracy of images are ensured.

In one possible example, the data enhancement processing includes elastic deformation; the performing of the elastic deformation on the image source to obtain the N first image data sets, including: superposing random distances in N directions on a raw pixel lattice of the image source to form N difference position matrices, wherein the N directions include at least positive and negative directions; calculating a gray value of a corresponding pixel point on each difference position matrix to obtain N enhanced pixel lattices; generating the N first image data sets according to the N enhanced pixel lattices, wherein the image data in each first image data set includes superposition information of pixel points.

In a specific implementation, superimposing positive and negative random distances on the image lattice of the image source to form a difference position matrix, and then calculating the gray value of the corresponding pixel at each difference position to form a new lattice can achieve the internal distortion of the image, and further performing rotation, distortion and translation operations etc. on the image.

It can be seen that in this example, because the medical imaging apparatus can perform elastic deformation processing on the image source, the execution efficiency of image processing is improved, and the refinement degree and accuracy of the medical imaging apparatus in performing medical image display is improved.

In one possible example, the performing of the preset processing on the N first image data sets to obtain the N second image data sets, including: performing data separation on the N first image data sets to obtain a separated data set, wherein the separated data set includes a plurality of separated data subsets, and image data in each separated data subset includes tissue identification information, and the tissue identification information is configured to identify a tissue to which the image data belongs, and the tissue includes at least one of the following: organs, tumors and blood vessels; performing data integration on the N first image data sets to obtain an integrated data set, wherein the integrated data set includes a plurality of types of image data of a plurality of tissues with a preset occupation mode; introducing the separated data set and the integrated data set into a VRDS AI 4D imaging data analyzer, and obtaining spatial position information of each image data meeting a gray matching condition by AI matching of medical image gray values and conversion calculation of the superposition information of the pixel points; generating the N second image data sets according to the spatial position information and the N first image data sets.

Wherein, the preset occupation mode refers to the preset combination of different tissues and organs, such as the combination of kidney and artery, the combination of kidney and tumor, etc.

In a specific implementation, performing data separation on the N first image data sets according to the tissue identification information, for example, the N first image data sets may be subjected to data separation into organ data subsets, tumor data subsets, blood vessel data subsets, and the like. Then, performing data integration on the N first image data sets, and image data meeting gray matching conditions and spatial position information of each image data are obtained from the integrated data sets by using the pre-stored medical image template matching models of the plurality of tissues and the superposition information of the pixel points, thereby generating N second image data sets.

It can be seen that in this example, because the medical imaging apparatus can generate N second image data sets based on the separation and integration of N first image data sets, AI matching of medical image gray values and conversion calculation of superposition information of the pixel points, the image data can be merged, consolidated and enhanced.

In one possible example, the AI matching of the medical image gray value is achieved by the following steps: invoking a pre-stored medical image template matching model of the plurality of tissues; introducing image data in the integrated data set into the medical image template matching model, and screening out image data meeting gray matching conditions.

Wherein, gray matching can determine the corresponding relationship of images by using some similarity measures, such as correlation function, covariance function, sum of squares of differences, absolute values of differences and extreme value of equal measure.

In a specific implementation, the image matching can be divided into several steps, such as image input, image preprocessing, matching feature extraction, image matching, and outputting of results. First, the image is preprocessed to extract its high-level feature values, and then according to the medical image template matching model, the image data that meet the gray matching conditions are screened out. The medical image template matching model can be constructed by various algorithms such as convolution neural network, and trained by medical sample data in advance to improve the accuracy of model prediction.

It can be seen that, in this example, because the medical imaging apparatus can obtain the image data meeting the gray matching conditions based on the pre-stored medical image template matching models of the plurality of tissues and the image data in the integrated data set, the quality of the medical image is improved, and the fineness and accuracy of the image are ensured.

In one possible example, the conversion calculation of the superposition information of the pixel points is achieved by the following operations: acquiring the superposition information of a currently processed pixel points, wherein the superposition information includes direction information and a random distance parameter; calculating spatial coordinates as spatial position information of the pixel points, according to the direction information and the random distance parameter.

Wherein, the conversion calculation of the superposition information of the pixel points is to calculate the spatial coordinates according to the direction information and the random distance parameter of the pixel points.

In a specific implementation, the superposition information data of the currently processed pixel point is acquired, and the obtained superposition information data is used, wherein the direction information and random distance parameters in the superposition information are obtained by superposition of at least two directions or at least two random distance parameters; and finally, spatial coordinates are calculated according to the direction information and the random distance parameters, and the spatial coordinates are the spatial position information of the pixel point.

It can be seen that in this example, because the medical imaging apparatus can calculate the spatial coordinates as the spatial position information of the pixel point based on the direction information of the pixel point and the random distance parameter, reflecting the spatial position attribute of the image data, the perfection of the data information of the image data and the accuracy and clarity of medical image imaging are improved.

In one possible example, the performing of the VRDS 4D medical image display according to the N second image data sets, including: screening target image data associated with a tissue from the N second image data sets as VRDS 4D imaging data according to the tissue associated with the preset occupation mode; outputting the VRDS 4D imaging data on a display device.

In a specific implementation, due to the spatial correlation between organs, blood vessels and other tissues, the distribution positions of image data of the same organ of human body in the N second image data sets are often interrelated, in order to shorten the screening time of image data and improve the data screening efficiency, when screening the first second image data set, the medical imaging apparatus traverses the whole data set in order from front to back, so as to screen out the first target image data, then, when screening the second second image data set, the position of the first target image data can be taken as the initial detection position of the current set, and the screening range can be reduced to the maximum range of the currently processed tissue (the tissue includes organs such as kidney, and the maximum range can be determined according to the volume of the organ, etc.), the processing operation from the third second image data set to the Nth second image data set is similar to the processing process of the second second image data set, so it is not repeated here. Therefore, the data screening efficiency and the image display efficiency can be significantly improved.

For example, when the tissues associated with the preset occupancy mode are hearts and blood vessels, the target image data associated with the hearts and blood vessels can be screened out from the N second image data according to the tissue identification information of the image data, and the target image data can be used as VRDS 4D imaging data for VRDS 4D imaging to display the images of the hearts and blood vessels. Wherein, the N second image data may include at least one of organs, tumors and blood vessels.

Furthermore, after outputting the VRDS 4D image on the display device, when detecting that the partial position for a certain tissue is selected, the image of the selected partial position of the tissue can be invoked, and operations such as zooming in, rotating, and observing the inner side can be executed.

It can be seen that in this example, because the medical imaging apparatus can screen out the tissue corresponding target data associated with the preset occupation mode based on the tissue associated with the preset occupation mode for VRDS 4D imaging data and display, the quality of medical images and the refinement degree and accuracy of medical image display by the medical imaging apparatus are improved.

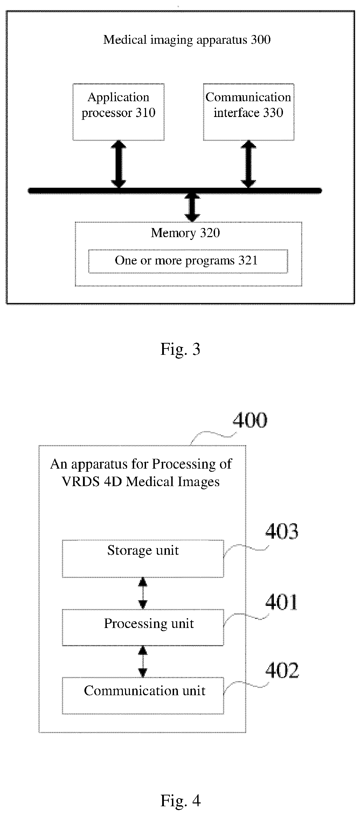

Consistent with the embodiments shown in Fig. 2, referring to Fig. 3, Fig. 3 is a schematic structural diagram of a medical imaging apparatus 300 provided by an embodiment of this application, as shown in the figure, the medical imaging apparatus 300 includes a processor 310, a memory 320, a communication interface 330 and one or more programs 321, wherein the one or more programs 321 are stored in the above memory 320 and configured to be executed by the above processor 310, and the one or more programs 321 include instructions for executing the following steps:
performing initial data analysis processing on a plurality of scanned images to obtain an image source including image features of a target site of a target user, wherein the image source includes Texture 2D/3D image volume data, and the plurality of scanned images are two-dimensional medical images collected by a medical device;
performing post-data processing on the image source to obtain N first image data sets, wherein a data amount of each first image data set is the same as that of the image source, and data in any two first image data sets are independent from each other, and N is a positive integer greater than 1;
performing preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and
performing VRDS 4D medical image display according to the N second image data sets.

As can be seen that in the embodiment of this application, first, the processing unit performs initial data analysis processing on a plurality of scanned images to obtain an image source including image features of a target site of a target user; second, performs post data processing on the image source to obtain N first image data sets, wherein a data amount of each first image data set is the same as that of the image source, and the data in any two first image data sets are independent from each other, and N is a positive integer greater than 1; third, performs preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and finally, performs VRDS 4D medical image display according to the N second image data sets. It can be seen that the medical imaging apparatus in this application can perform data optimization on the raw scanned image in the data volume dimension and spatial dimension, so as to achieve VRDS 4D medical image display, that is, to present 3D images outside and inside the tissue with real spatial structure characteristics, and to improve the refinement degree and accuracy of the medical imaging apparatus in performing medical image display.

In one possible example, in the aspect of the performing of the post-data processing on the image source to obtain the N first image data sets, the instructions in the program are specifically configured to perform the following operation: performing data enhancement processing on the image source to obtain N first image data sets, wherein the data enhancement processing includes at least one of the following: rotation, scaling, translation, shearing, mirroring and elastic deformation.

In one possible example, in the aspect of the data enhancement processing includes elastic deformation; the performing of the elastic deformation on the image source to obtain the N first image data sets, the instructions in the program are specifically configured to perform the following operation: superposing random distances in N directions on a raw pixel lattice of the image source to form N difference position matrices, wherein the N directions include at least positive and negative directions; calculating a gray value of a corresponding pixel point on each difference position matrix to obtain N enhanced pixel lattices; generating the N first image data sets according to the N enhanced pixel lattices, wherein the image data in each first image data set includes superposition information of pixel points.

In one possible example, in the aspect of the performing of the preset processing on the N first image data sets to obtain the N second image data sets, the instructions in the program are specifically configured to perform the following operation: performing data separation on the N first image data sets to obtain a separated data set, wherein the separated data set includes a plurality of separated data subsets, and image data in each separated data subset includes tissue identification information, and the tissue identification information is configured to identify a tissue to which the image data belongs, and the tissue includes at least one of the following: organs, tumors and blood vessels; performing data integration on the N first image data sets to obtain an integrated data set, wherein the integrated data set includes a plurality of types of image data of a plurality of tissues with a preset occupation mode; introducing the separated data set and the integrated data set into a VRDS AI 4D imaging data analyzer, and obtaining spatial position information of each image data meeting a gray matching condition by AI matching of medical image gray values and conversion calculation of the superposition information of the pixel points; generating the N second image data sets according to the spatial position information and the N first image data sets.

In one possible example, in the aspect of the AI matching of the medical image gray value, the instructions in the program are specifically configured to perform the following operation: invoking a pre-stored medical image template matching model of the plurality of tissues; introducing image data in the integrated data set into the medical image template matching model, and screening out image data meeting gray matching conditions.

In one possible example, in the aspect of the conversion calculation of the superposition information of the pixel points, the instructions in the program are specifically configured to perform the following operation: acquiring the superposition information of a currently processed pixel points, wherein the superposition information includes direction information and a random distance parameter; calculating spatial coordinates as spatial position information of the pixel points, according to the direction information and the random distance parameter.

In one possible example, in the aspect of the performing of the VRDS 4D medical image display according to the N second image data sets, the instructions in the program are specifically configured to perform the following operation: screening target image data associated with a tissue from the N second image data sets as VRDS 4D imaging data according to the tissue associated with the preset occupation mode; outputting the VRDS 4D imaging data on a display device.

The above mainly introduces the solution of the embodiment of this application from the perspective of the execution process on the method side. It can be understood that in order to achieve the above functions, the medical imaging apparatus includes corresponding hardware structures and/or software modules for performing various functions. It should be easy for the skill person in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 for processing of VRDS 4D medical images involved in the embodiment of this application. The apparatus 400 for processing of VRDS 4D medical images is applied to a medical imaging apparatus, the apparatus 400 for processing of VRDS 4D medical image includes a processing unit 401 and a communication unit 402.

The processing unit 401 is configured to: perform initial data analysis processing on a plurality of scanned images to obtain an image source including image features of a target site of a target user, wherein the image source includes Texture 2D/3D image volume data, and the plurality of scanned images are two-dimensional medical images collected by a medical device; perform post data processing on the image source to obtain N first image data sets, wherein a data amount of each first image data set is the same as that of the image source, and the data in any two first image data sets are independent from each other, and N is a positive integer greater than 1; perform preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and perform VRDS 4D medical image display according to the N second image data sets through the communication unit 402.

The apparatus 400 for processing of VRDS 4D medical images further includes a storage unit 403, wherein the processing unit 401 can be a processor, the communication unit 402 can be a transceiver, and the storage unit can be a memory.

As can be seen that in the embodiment of this application, first, the processing unit performs initial data analysis processing on a plurality of scanned images to obtain an image source including image features of a target site of a target user; second, performs post data processing on the image source to obtain N first image data sets, wherein a data amount of each first image data set is the same as that of the image source, and the data in any two first image data sets are independent from each other, and N is a positive integer greater than 1; third, performs preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect a spatial position attribute of image data; and finally, performs VRDS 4D medical image display according to the N second image data sets. It can be seen that the medical imaging apparatus in this application can perform data optimization on the raw scanned image in the data volume dimension and spatial dimension, so as to achieve VRDS 4D medical image display, that is, to present 3D images outside and inside the tissue with real spatial structure characteristics, and to improve the refinement degree and accuracy of the medical imaging apparatus in performing medical image display.

In one possible example, in the aspect of the performing of the post-data processing on the image source to obtain the N first image data sets, the processing unit 401 is specifically configured to: perform data enhancement processing on the image source to obtain N first image data sets, wherein the data enhancement processing includes at least one of the following: rotation, scaling, translation, shearing, mirroring and elastic deformation.

In one possible example, in the aspect of the data enhancement processing includes elastic deformation; the performing of the elastic deformation on the image source to obtain the N first image data sets, the processing unit 401 is specifically configured to: superpose random distances in N directions on a raw pixel lattice of the image source to form N difference position matrices, wherein the N directions include at least positive and negative directions; calculate a gray value of a corresponding pixel point on each difference position matrix to obtain N enhanced pixel lattices; generate the N first image data sets according to the N enhanced pixel lattices, wherein the image data in each first image data set includes superposition information of pixel points.

In one possible example, in the aspect of the performing of the preset processing on the N first image data sets to obtain the N second image data sets, the processing unit 401 is specifically configured to: perform data separation on the N first image data sets to obtain a separated data set, wherein the separated data set includes a plurality of separated data subsets, and image data in each separated data subset includes tissue identification information, and the tissue identification information is configured to identify a tissue to which the image data belongs, and the tissue includes at least one of the following: organs, tumors and blood vessels; perform data integration on the N first image data sets to obtain an integrated data set, wherein the integrated data set includes a plurality of types of image data of a plurality of tissues with a preset occupation mode; introduce the separated data set and the integrated data set into a VRDS AI 4D imaging data analyzer, and obtain spatial position information of each image data meeting a gray matching condition by AI matching of medical image gray values and conversion calculation of the superposition information of the pixel points; generate the N second image data sets according to the spatial position information and the N first image data sets.

In one possible example, in the aspect of the AI matching of the medical image gray value, the processing unit 401 is specifically configured to: invoke a pre-stored medical image template matching model of the plurality of tissues; introduce image data in the integrated data set into the medical image template matching model, and screen out image data meeting gray matching conditions.

In one possible example, in the aspect of the conversion calculation of the superposition information of the pixel points, the processing unit is 401 specifically configured to: acquire the superposition information of a currently processed pixel points, wherein the superposition information includes direction information and a random distance parameter; calculate spatial coordinates as spatial position information of the pixel points, according to the direction information and the random distance parameter.

In one possible example, in the aspect of the performing of the VRDS 4D medical image display according to the N second image data sets, the processing unit is specifically configured to: screen target image data associated with a tissue from the N second image data sets as VRDS 4D imaging data according to the tissue associated with the preset occupation mode; output the VRDS 4D imaging data on a display device.

Embodiments of this application further provide a computer storage medium, wherein the computer storage medium stores a computer program for electronic data exchange, the computer program causes a computer to execute part or all of the steps of any method as recorded in the above method embodiment, and the above computer includes a medical imaging apparatus.

Embodiments of this application further provide a computer program product, the above computer program product includes a non-transitory computer-readable storage medium in which a computer program is stored, the above computer program is operable to cause a computer to execute part or all of the steps of any method as recorded in the above method embodiments. The computer program product can be a software installation package, and the above computer includes a medical imaging apparatus.

It should be noted that, for brevity of description, the foregoing method embodiments are described as a series of movement combinations. However, the skilled person in the art should learn that this application is not limited by the movement sequence, because according to this application, some steps can be performed in other order or simultaneously. Besides, it also should be known by the skill person in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer-readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM), a removable hard disk, a magnetic disk, or an optical disc.

Those skilled in the art may understand that all or some of the steps in various methods of the above embodiments can be completed by instructing related hardware through programs, which can be stored in a computer-readable memory, which can include: flash disks, a read-only memory (ROM), a random access memory (RAM), disks or optical disks, etc.

The embodiments of this application are described in detail above, and the principles and implementation way of this application are explained by specific examples. The above embodiments are only used to help understand the method and its core ideas of this application; at the same time, according to the idea of this application, there will be some changes in the specific implementation way and application scope for the ordinary skill person in the art. As above, the contents of this specification should not be construed as limitations of this application.

## Claims

1. A method for processing of Virtual Reality Doctor system (VRDS) 4D medical images, **characterized in that** the method is applied to medical imaging apparatus; and the method comprises:
performing initial data analysis processing on a plurality of scanned images to obtain an image source including image features of a target site of a target user, wherein the image source comprises Texture 2D/3D image volume data, and the plurality of scanned images are two-dimensional medical images collected by medical device;
performing post-data processing on the image source to obtain N first image data sets, wherein the data amount of each first image data set is the same as that of the image source, and the data in any two first image data sets are independent from each other, and N is a positive integer greater than 1;
performing preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to reflect spatial position attributes of image data; and
performing VRDS 4D medical images display according to the N second image data sets.

2. The method according to claim 1, **characterized in that** the performing of the post-data processing on the image source to obtain the N first image data sets comprises:
performing data enhancement processing on the image source to obtain the N first image data sets, wherein the data enhancement processing comprises at least one of the following:
rotation, scaling, translation, shearing, mirroring and elastic deformation.

3. The method according to claim 1, **characterized in that** the data enhancement processing comprises elastic deformation; the performing of the elastic deformation on the image source to obtain the N first image data sets comprises:
superposing random distances in N directions on a raw pixel lattice of the image source to form N difference position matrices, wherein the N directions comprise at least positive and negative directions;
calculating a gray value of a corresponding pixel point on each difference position matrix to obtain N enhanced pixel lattices;
generating the N first image data sets according to the N enhanced pixel lattices, wherein the image data in each first image data set comprises superposition information of pixel points.

4. The method according to claim 3, **characterized in that** the performing preset processing on the N first image data sets to obtain the N second image data sets comprises:
performing data separation on the N first image data sets to obtain a separated data set, wherein the separated data set comprises a plurality of separated data subsets, and the image data in each separated data subset comprises tissue identification information, and the tissue identification information is configured to identify the tissue to which the image data belongs, and the tissue comprises at least one of the following organs, tumors and blood vessels;
performing data integration on the N first image data sets to obtain an integrated data set, wherein the integrated data set comprises a plurality of types of image data of a plurality of tissues with preset occupation modes;
introducing the separated data set and the integrated data set into a VRDS AI 4D imaging data analyzer, and obtaining a spatial position information of each image data meeting a gray matching condition by AI matching of medical image gray values and conversion calculation of the superposition information of the pixel points;
generating the N second image data sets according to the spatial position information and the N first image data sets.

5. The method according to claim 4, **characterized in that** the AI matching of the medical image gray value is achieved by the following steps: invoking a pre-stored medical image template matching model of the plurality of tissues; introducing the image data in the integrated data set into the medical image template matching model, and screening out the image data meeting the gray matching conditions.

6. The method according to claim 4 or 5, **characterized in that** the conversion calculation of the superposition information of the pixel points is achieved by the following operations:
acquiring the superposition information of a currently processed pixel points, wherein the superposition information comprises direction information and random distance parameters;
calculating spatial coordinates as spatial position information of the pixel points, according to the direction information and the random distance parameter, .

7. The method according to claim 6, **characterized in that** the conversion calculation of the superposition information of the pixel points is to calculate the spatial coordinates according to the direction information and random distance parameters of the pixel points.

8. The method according to any one of claims 1-7, **characterized in that** the performing of the VRDS 4D medical image display according to the N second image data sets comprises:
screening target image data associated with the tissue from the N second image data sets as VRDS 4D imaging data according to the tissue associated with the preset occupation mode;
outputting the VRDS 4D imaging data on a display device.

9. The method according to claim 1, **characterized in that** after the performing of the VRDS 4D medical image display according to the N second image data sets, the method further comprises:
detecting a target position selection instruction for a target tissue
taking an image of the selected target position according to the selection instruction, and performing preset operation on the image, wherein the preset operation comprises at least one of the following: enlarging, rotating or observing the inner side.

10. An apparatus for processing of VRDS 4D medical images, **characterized in that** the apparatus is applied to a medical imaging apparatus; the VRDS 4D medical image processing apparatus comprises a processing unit and a communication unit, wherein,
the processing unit is configured to: perform initial data analysis processing on a plurality of scanned images to obtain an image source including image features of a target site of a target user, wherein the image source comprises Texture 2D/3D image volume data, and the plurality of scanned images are two-dimensional medical images collected by medical device; perform post-data processing on the image source to obtain N first image data sets, wherein the data amount of each first image data set is the same as that of the image source, and the data in any two first image data sets are independent from each other, and N is a positive integer greater than 1; perform preset processing on the N first image data sets to obtain N second image data sets, wherein the N first image data sets are in a one-to-one correspondence with the N second image data sets, and each second image data set is added with spatial position information relative to the corresponding first image data sets, and the spatial position information is configured to: reflect spatial position attributes of image data; and perform VRDS 4D medical images display according to the N second image data sets through the communication unit.

11. The apparatus according to claim 10, **characterized in that** in the aspect of the performing of the post-data processing on the image source to obtain the N first image data sets, the processing unit is specifically configured to: perform data enhancement processing on the image source to obtain the N first image data sets, wherein the data enhancement processing comprises at least one of the following: rotation, scaling, translation, shearing, mirroring and elastic deformation.

12. The apparatus according to claim 10, **characterized in that** the data enhancement processing comprises elastic deformation; in the aspect of the performing of the elastic deformation on the image source to obtain the N first image data sets, the processing unit is specifically configured to: superpose random distances in N directions on a raw pixel lattice of the image source to form N difference position matrices, wherein the N directions comprise at least positive and negative directions; calculate a gray value of a corresponding pixel point on each difference position matrix to obtain N enhanced pixel lattices; generate the N first image data sets according to the N enhanced pixel lattices, wherein the image data in each first image data set comprises superposition information of pixel points.

13. The apparatus according to claim 12, **characterized in that** in the aspect of the performing preset processing on the N first image data sets to obtain the N second image data sets, the processing unit is specifically configured to: perform data separation on the N first image data sets to obtain a separated data set, wherein the separated data set comprises a plurality of separated data subsets, and the image data in each separated data subset comprises tissue identification information, and the tissue identification information is configured to identify the tissue to which the image data belongs, and the tissue comprises at least one of the following organs, tumors and blood vessels; perform data integration on the N first image data sets to obtain an integrated data set, wherein the integrated data set comprises a plurality of types of image data of a plurality of tissues with preset occupation modes; introduce the separated data set and the integrated data set into a VRDS AI 4D imaging data analyzer, and obtain a spatial position information of each image data meeting a gray matching condition by AI matching of medical image gray values and conversion calculation of the superposition information of the pixel points; generate the N second image data sets according to the spatial position information and the N first image data sets.

14. The apparatus according to claim 13, **characterized in that** the AI matching of the medical image gray value is achieved by the following steps: invoking a pre-stored medical image template matching model of the plurality of tissues; introducing the image data in the integrated data set into the medical image template matching model, and screening out the image data meeting the gray matching conditions.

15. The apparatus according to claim 13 or 14, **characterized in that** the conversion calculation of the superposition information of the pixel points is achieved by the following operations: acquiring the superposition information of a currently processed pixel points, wherein the superposition information comprises direction information and random distance parameters; calculating spatial coordinates as spatial position information of the pixel points, according to the direction information and the random distance parameter

16. The apparatus according to claim 15, **characterized in that** the conversion calculation of the superposition information of the pixel points is to calculate the spatial coordinates according to the direction information and random distance parameters of the pixel points.

17. The apparatus according to any one of claims 10-16, **characterized in that** in the aspect of the performing of the VRDS 4D medical image display according to the N second image data sets, the communication unit is specifically configured to: screen target image data associated with the tissue from the N second image data sets as VRDS 4D imaging data according to the tissue associated with the preset occupation mode; and output the VRDS 4D imaging data on a display device.

18. The apparatus according to claim 10, **characterized in that** after the performing of the VRDS 4D medical image display according to the N second image data sets, the processing unit is further specifically configured to: detect a target position selection instruction for a target tissue; take an image of the selected target position according to the selection instruction, and perform preset operation on the image, wherein the preset operation comprises at least one of the following: enlarging, rotating or observing the inner side.

19. A medical imaging apparatus, **characterized in that** the apparatus comprises a processor, a memory, a communication interface, and one or more programs, the one or more programs are stored in the memory and configured to be executed by the processor, and the programs comprise instructions for executing the steps in the method according to any one of claims 1-9.

20. A computer-readable storage medium, **characterized in that** the computer-readable storage medium stores a computer program for electronic data exchange, and wherein the computer program causes a computer to execute the method according to any one of claims 1-9.
